# FASCICULE DE BREVET EUROPEEN

(11) **EP 1 029 587 B1**
(45) Date de publication et mention de la délivrance du brevet: **08.06.2005**
(21) Numéro de dépôt: 99403272.0
(22) Date de dépôt: 23.12.1999
(51) Int. Cl.: B01J 13/04, A61K 7/00, A61K 9/51

(54) **Nanocapsules à base de poly(alkylène adipate), leur procédé de préparation et compositions cosmétiques ou dermatologiques les contenant**
Nanokapseln auf basis von poly(alkylène adipate),ihren Verfahren, und diese enthaltende kosmetische oder dermatologische Zusammensetzungen
Nanocapsules based on poly(alkylene adipate), their fabrication process, cosmetic and dermatological compositions containing them

(30) Priorité: 29.12.1998 FR 9816554
(43) Date de publication de la demande: 23.08.2000
(73) Titulaire: L'OREAL, 75008 Paris (FR)
(72) Inventeur: Simonnet, Jean-Thierry, 75011 Paris (FR); Richart, Pascal, 75013 Paris (FR); Biatry, Bruno, 94300 Vincennes (FR)
(74) Mandataire: Casalonga, Axel

(56) Documents cités:
- EP-A- 0 254 447
- EP-A- 0 274 961
- EP-A- 0 447 318
- FR-A- 2 681 248
- DATABASE WPI Section Ch, Week 8917 Derwent Publications Ltd., London, GB; Class A96, AN 89-126027 XP002116511 & JP 01 071823 A (ROHTO PHARM CO LTD), 16 mars 1989 (1989-03-16)

## Description

La présente invention concerne des nanocapsules à base de polyesters de type poly(alkylène adipate), leur procédé de préparation ainsi que des compositions cosmétiques ou dermatologiques les contenant.

L'encapsulation ou l'absorption de principes actifs liphophiles dans des particules de dimensions submicroniques est connue depuis plusieurs années et est largement utilisée en particulier dans les domaines cosmétologique et dermatologique. En effet, ces particules appelées nanoparticules sont capables de traverser les couches superficielles du *stratum corneum* et de pénétrer dans les couches supérieures de l'épiderme vivant pour y libérer le principe actif. Cette pénétration dans des couches plus profondes élargit l'espace d'action des principes actifs et les met à l'abri d'une élimination rapide par simple frottement.

Le terme de "nanoparticules" englobe principalement deux systèmes différents : des "nanosphères" constituées d'une matrice polymérique poreuse dans laquelle le principe actif est absorbé et/ou adsorbé, ainsi que des "nanocapsules" ayant une structure de type noyau-enveloppe, c'est-à-dire une structure constituée d'un coeur lipidique formant ou contenant le principe actif, lequel coeur est encapsulé dans une enveloppe protectrice continue insoluble dans l'eau. La présente invention concerne uniquement ce deuxième type vésiculaire de nanoparticules, c'est-à-dire des nanocapsules à noyau lipidique entouré d'une membrane polymère.

L'encapsulation de principes actifs dans des capsules de taille submicronique permet, il est vrai, de transporter les molécules actives plus profondément dans la peau mais elle n'assure pas toujours - contrairement à ce que pourrait laisser penser cette structure "protectrice" - une stabilité suffisante du principe actif vis-à-vis des conditions physico-chimiques environnantes.

Le problème de l'instabilité du principe actif se pose en particulier pour des substances sensibles à l'oxydation, à la lumière, à des températures élevées et/ou à des pH acides ou basiques. Une telle substance très utilisée en cosmétique est par exemple le rétinol (vitamine A) qui est sensible à l'oxydation en particulier à pH acide.

Une approche pour stabiliser le rétinol a consisté à ajouter aux compositions le renfermant des antioxydants liphophiles et des chélatants et à ajuster le pH de ces compositions à une valeur comprise entre 5 et 10 (WO 96/31194).

La demanderesse a découvert que l'encapsulation dans des nanocapsules à base d'un type de polymère particulier permettait d'améliorer de manière spectaculaire la stabilité du rétinol et ceci notamment en l'absence d'agents anti-oxydants.

Les polymères permettant d'obtenir un tel effet favorable sont des polyesters de type poly(alkylène adipate) décrits plus en détail ci-dessous.

Ainsi, l'encapsulation du rétinol dans des nanocapsules ayant une enveloppe formée par des polyesters de type poly(alkylène adipate) confère à cette molécule active une stabilité satisfaisante, à savoir une perte d'activité de seulement 5 à 10 % après 2 mois de conservation à 45 °C, alors que, dans des conditions équivalentes, cette même molécule encapsulée dans d'autres polymères couramment utilisés pour la nanoencapsulation (par exemple la polycaprolactone ou les dérivés de cellulose) présente une perte d'activité supérieure à 20 %.

L'invention a donc pour objet des nanocapsules constituées
- d'un coeur lipidique formant ou contenant un principe actif lipophile et
- d'une enveloppe polymérique continue insoluble dans l'eau comprenant au moins un polyester de type poly(alkylène adipate).

Elle a également pour objet des compositions cosmétiques et/ou dermatologiques contenant lesdites nanocapsules à base de poly(alkylène adipate).

L'invention a en outre pour objet un procédé de préparation des nanocapsules à base de poly(alkylène adipate) ci-dessus.

D'autres objets apparaîtront à la lecture de la description et des exemples qui suivront.

Les polyesters utilisables pour former l'enveloppe des nanocapsules sont des polymères obtenus par polycondensation d'un acide dicarboxylique aliphatique, à savoir l'acide adipique (acide hexane-1,6-dioïque), et d'un ou de plusieurs diols et, éventuellement, d'une faible proportion de triols.

Le terme de *poly(alkylène adipate)* utilisé dans la présente demande pour désigner les polyesters formant l'enveloppe des nanocapsules englobe à la fois les *homopolymères* d'acide adipique et d'un alcane-diol et les *copolymères* de type poly(ester éther), linéaires ou ramifiés, obtenus à partir d'acide adipique et d'un ou de plusieurs alcane-diols et/ou éthers-diols et/ou triols.

Les alcane-diols utilisés pour la préparation desdits poly(alkylène adipate) sont des alcane-diols en C₂₋₆ à chaîne linéaire ou ramifiée choisis parmi l'éthylèneglycol, le propylèneglycol, le 1,3-propanediol, le 1,4-butanediol, le 1,5-pentanediol, le 1,6-hexanediol et le néopentylglycol.

Les éther-diols sont des di-, tri- ou tétra-(alkylène en C₂₋₄)-glycols tels que le diéthylèneglycol, le triéthylèneglycol, le tétraéthylèneglycol, le dipropylèneglycol, le tripropylèneglycol, le tétrapropylèneglycol ou le dibutylèneglycol, le tributylèneglycol ou le tétrabutylèneglycol.

Comme indiqué ci-dessus, les polyesters de type poly(alkylène adipate) utilisés pour la préparation des nanocapsules de l'invention peuvent également contenir un nombre limité de motifs de ramification dérivés de triols.

Les triols utilisés sont généralement choisis parmi le glycérol, le triméthyloléthane et le triméthylolpropane.

La fraction des motifs de ramification dérivés des triols ci-dessus n'excède généralement pas 5 % en moles par rapport à l'ensemble des motifs dérivés de diols et de triols.

Selon un mode de réalisation préféré de la présente invention, l'enveloppe des nanocapsules est formée par un poly(éthylène adipate) ou un poly(butylène adipate).

Les poly(alkylène adipate) utilisés dans la présente invention ont une masse molaire moyenne en poids (mesurée par chromatographie de perméation de gel) comprise entre 2000 et 50 000, de préférence entre 5 000 et 15 000.

Cette gamme des masses molaires est limitée d'un côté, pour les faibles masses, par une teneur trop importante en oligomères et monomères résiduels, et pour les masses importantes, par un coût de production rédhibitoire.

Les poly(alkylène adipate) utilisés dans la présente invention sont connus et peuvent être préparés selon des procédés connus.

Toute une gamme de produits de différentes compositions chimiques et de différentes masses est commercialisée sous la dénomination FOMREZ® par la société WITCO. La société SCIENTIFIC POLYMER PRODUCTS commercialise sous la dénomination POLY(ETHY-LENE)ADIPATE® un poly(éthylène adipate) d'une masse molaire moyenne en poids (déterminée par CPG) d'environ 10 000.

Les polyesters de type poly(alkylène adipate) décrits ci-dessus sont utilisés pour préparer des nanocapsules constituées d'un coeur lipidique formant ou contenant un principe actif lipophile, entouré d'une enveloppe formée par ces polymères.

Le procédé de préparation de nanocapsules utilisé de préférence par la demanderesse est celui décrit dans EP-A-0 274 961 et comprend les étapes consistant
- à dissoudre le polymère, la phase lipidique formant ou contenant le principe actif et éventuellement un agent tensioactif jouant le rôle d'agent d'enrobage dans un solvant organique approprié, c'est-à-dire miscible avec l'eau, non toxique et plus volatil que l'eau (généralement de l'acétone et/ou un alcool inférieur),
- à préparer une solution d'un agent tensioactif approprié dans de l'eau (non-solvant du polymère et de la phase lipidique),
- à verser la phase organique dans la phase aqueuse tout en agitant modérément celle-ci, ce qui aboutit à la formation spontanée d'une émulsion de nanocapsules,
- puis à évaporer la phase organique et, éventuellement, une partie de la phase aqueuse, pour obtenir une suspension concentrée de nanocapsules dans une phase aqueuse.

Ce procédé de préparation implique généralement le chauffage de la phase organique et/ou de la phase aqueuse à des températures comprises entre 35 et 70 °C. Les poly(alkylène adipate) utilisés dans la présente invention permettent de réaliser ce procédé à température ambiante ce qui constitue un avantage important en particulier pour des substances actives thermosensibles telles que le rétinol.

L'agent tensioactif dissous dans la phase aqueuse sert principalement à maîtriser la taille des nanocapsules. Il assure en effet la stabilité des nanocapsules dans l'émulsion résultant du versement de la phase acétonique dans la phase aqueuse et prévient leur coalescence.

On peut utiliser n'importe quel agent tensioactif à caractère hydrophile, qu'il soit non-ionique, anionique ou cationique. On peut citer à titre d'exemple le laurylsulfate de sodium, les composés d'ammonium quaternaire, les monoesters de sorbitanne polyoxyéthylénés ou non, les éthers d'alcools gras et de polyoxyéthylèneglycol, les condensats d'oxyde d'éthylène et d'oxyde de propylène comme le produit PLURONIC® F-68 vendu par la société BASF ou les phospholipides tels que la lécithine.

Le rapport pondéral de l'agent tensioactif aux matériaux constitutifs des nanocapsules est avantageusement compris entre 0,01 et 0,5 et de préférence voisin de 0,2.

Il est souvent souhaitable ou nécessaire de pourvoir les nanocapsules d'un enrobage dit "lamellaire". Il s'agit d'une structure organisée en un ou plusieurs feuillet(s) lipidique(s) constitué(s) chacun d'une bicouche de molécules amphiphiles semblable à celle des membranes biologiques.

Cet enrobage, outre sa fonction d'ajustement de la taille des nanocapsules, améliore l'étanchéité des nanocapsules vis-à-vis d'une fuite du principe actif vers une autre phase lipidique de la composition.

Les agents d'enrobage sont des agents tensioactifs à caractère hydrophobe, solubles dans la phase organique utilisée dans le procédé décrit ci-dessus et qui sont capables, en présence d'eau, de former les doubles couches lipidiques décrites ci-dessus. Dans le procédé d'encapsulation de principes actifs utilisé par la demanderesse, cet agent d'enrobage est dissous dans la phase organique (acétonique/alcoolique) contenant le polymère et la phase lipidique.

On peut citer à titre d'exemple de tels agents d'enrobage les phospholipides tels que la lécithine selon la demande EP-A-447 318, certains polycondensats d'oxyde d'éthylène et d'oxyde de propylène comme les produits vendus sous la dénomination PLURONIC® par la société BASF tels que PLURONIC® L121 ou sous la dénomination SYNPERONIC® par la société ICI, ou certains agents tensioactifs siliconés, tels que ceux décrits dans les documents US-A-5 364 633 et US-A-5 411 744 et utilisés dans la demande de brevet FR-A-2 742 677, par exemple ceux vendus par la société DOW CORNING sous les dénominations DC 5329, DC 7439-146, DC 2-5695 et Q4-3667.

La taille moyenne des nanocapsules à base de poly(alkylène adipate) ainsi obtenues est avantageusement comprise entre 50 et 800 nm, de préférence entre 100 et 300 nm. La détermination de cette taille est réalisée par exemple à l'aide d'un granulomètre à laser (modèle Amtech BI 90 de la société Broohaven Instrument).

Les nanoencapsules de la présente invention peuvent contenir toutes sortes de principes actifs cosmétiques ou dermatologiques liphophiles.

On peut citer à titre d'exemple les agents émollients, les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les piégeurs de radicaux libres, les agents hydratants, les vitamines et d'autres composés lipophiles similaires.

Selon la présente invention, le principe actif lipophile encapsulé est de préférence un principe actif lipophile sensible aux conditions physico-chimiques environnantes telles que la température, le pH, la lumière ou la présence d'agents oxydants.

On peut citer à titre d'exemples de principes actifs lipophiles préférés les vitamines telles que la vitamine A (rétinol) ou des esters de celle-ci, la vitamine E ou des esters de celle-ci tels que l'acétate de tocophérol, la vitamine D ou des dérivés de celle-ci et la vitamine F ou des dérivés de celle-ci, les carotènes tels que le β-carotène et les dérivés de ceux-ci tels que le lycopène, et l'acide salicylique ou ses dérivés, notamment ceux décrits dans les documents FR-A-2 581 542, EP-A-378 936 et EP-A-570230, en particulier les acides n-octanoyl-5-salicylique, n-décanoyl-5-salicylique, n-dodécanoyl-5-salicylique, n-octyl-5-salicylique, n-heptyloxy-5-salicylique et n-heptyloxy-4-salicylique.

On a obtenu d'excellents résultats notamment pour l'encapsulation du rétinol (vitamine A), molécule très sensible à l'oxydation à pH acide, ainsi que pour les esters en C₁₋₃₀, plus particulièrement en C₁₋₆, de celui-ci, tels que l'acétate de rétinol et le propionate de rétinol.

La présente invention a également pour objet des compositions cosmétiques ou dermatologiques contenant, dans un support physiologiquement acceptable, les nanocapsules à base de poly(alkylène adipate) décrites ci-dessus.

La fraction que représentent les nanocapsules dans les compositions cosmétiques ou dermatologiques de la présente invention est généralement comprise entre 0,1 et 30 % en poids et de préférence entre 0,5 et 15 % en poids rapporté au poids total de la composition.

Les compositions peuvent contenir, en plus des nanocapsules et de la phase aqueuse, des adjuvants cosmétiques et/ou pharmaceutiques connus tels que des corps gras, de la vaseline, des agents régulateurs de pH, des conservateurs, des agents épaississants, des colorants ou des parfums.

Bien entendu, l'homme de métier veillera à choisir ce ou ces éventuels composés supplémentaires et leur quantité de manière à ce que les propriétés avantageuses attachées intrinsèquement à la composition cosmétique ou dermatologique conforme à l'invention ne soient pas, ou substantiellement pas, altérées par la ou les adjonctions envisagées.

Les compositions selon l'invention peuvent se présenter par exemple sous forme de sérum, de lotion, de gel aqueux, hydroalcoolique ou huileux, d'émulsion eau-dans-huile ou huile-dans-eau ou encore sous forme de dispersions aqueuses de vésicules lipidiques constituées de lipides ioniques ou non-ioniques ou d'un mélange de ceux-ci, lesquelles vésicules renferment ou non une phase huileuse.

Les exemples, donnés ci-après à titre purement illustratif et non limitatif, permettront de mieux comprendre l'invention.

### Exemple 1

### Préparation de nanocapsules à base de poly(éthylène adipate)

Dans un ballon en verre ambré d'une capacité de 500 ml, on dissout, sous atmosphère inerte, à température ambiante et sous agitation
- 1 g de poly(éthylène adipate) de la société SCIENTIFIC POLYMER PRODUCTS et
- 1g de Pluronic® L121 commercialisé par la société BASF dans 150 ml d'acétone.

D'autre part, dans un ballon en verre ambré d'une capacité de 250 ml, on dissout sous atmosphère inerte et lumière inactinique, à température ambiante et sous agitation 5 g de triglycérides d'acide caprique et d'acide caprylique (phase lipidique) contenant 10 % de rétinol dans 50 ml d'acétone.

Cette dernière solution est ajoutée au contenu du ballon de 500 ml.

Dans un ballon en verre ambré d'une capacité de 1 1, on dissout 0,5 g d'un agent tensioactif non-ionique (polycondensat triséquencé d'oxyde d'éthylène et d'oxyde de propylène commercialisé sous la dénomination PLURONIC® F68 par la société BASF) dans 300 g d'eau distillée sous atmosphère inerte et à température ambiante.

On verse la phase acétonique dans la phase aqueuse en maintenant l'agitation.

On évapore ensuite dans un évaporateur rotatif l'acétone et une partie de l'eau jusqu'à un volume final de 100 ml.

Cette suspension aqueuse contient des nanocapsules ayant un diamètre moyen de 250 nm.

### Exemple 2

### Préparation de nanocapsules à base de poly(butylène adipate)

On procède de la manière décrite dans l'Exemple 1 mais en remplaçant le poly(éthylène adipate) par une quantité identique en poids de poly(butylène adipate) fourni par la société ALDRICH.

On obtient une suspension aqueuse de nanocapsules à base de poly(butylène adipate) ayant un diamètre moyen de 270 nm.

### Exemple 3

### Essais de stabilité du rétinol encapsulé dans différents polymè res

On compare la stabilité du rétinol enfermé dans les nanocapsules à base de poly(éthylène adipate) (obtenues dans l'Exemple 1) à la stabilité de ce principe actif dans des nanocapsules à base de polymères connus qui sont la polycaprolactone et l'acétobutyrate de cellulose, enrobées d'un agent tensioactif non ionique.

Les nanocapsules contenant le rétinol sont conservées sous forme de suspension aqueuse pendant deux mois à 45 °C en conditionnements clos et étanches à la lumière et aux gaz. Au bout de cette période de conservation, on évalue la perte en principe actif (rétinol) par HPLC avec une détection à 325 nm.

Les résultats obtenus sont rassemblés dans le tableau suivant.

| polymère | poly(éthylène adipate) | poly(caprolactone) | acétobutyrate de cellulose |
|---|---|---|---|
| masse molaire du polymère | 10 000 | 50 000 | 30 000 |
| P_{f} du polymère (en °C) | 55 | 58 - 60 | 155 - 165 |
| agent d'enrobage | tensioactif non ionique* | tensioactif non ionique* | tensioactif non ionique* |
| diamètre moyen des nanocapsules | 243 nm | 266 nm | 213 nm |
| pH de la composition | 8,7 | 6,4 | 6,1 |
| perte de principe actif après 2 mois à 45 °C | 6 % | 28 % | 21 % |

| | | | |
|---|---|---|---|
| * Pluronic® L121 commercialisé par la société BASF. | | | |

Ces résultats montrent que l'utilisation du poly(éthylène adipate) pour la préparation de nanocapsules améliore de manière significative la stabilité à l'oxydation du rétinol encapsulé.

## Revendications

1. Nanocapsules constituées
- d'un coeur lipidique formant ou contenant un principe actif lipophile et
- d'une enveloppe polymérique continue insoluble dans l'eau, **caractérisées par le fait que** ladite enveloppe polymérique comprend au moins un polyester linéaire ou ramifié du type poly(alkylène adipate) comportant des motifs dérivés d'acide adipique et des motifs dérivés d'un ou de plusieurs alcane-diols et/ou d'un ou de plusieurs éther-diols et/ou d'un ou de plusieurs triols.

2. Nanocapsules selon la revendication 1, **caractérisées par le fait que** les alcane-diols sont des alcane-diols en C₂₋₆ à chaîne linéaire ou ramifiée choisis parmi l'éthylèneglycol, le propylèneglycol, le 1,3-propanediol, le 1,4-butanediol, le 1,5-pentanediol, le 1,6-hexanediol et le riéopentylglycol.

3. Nanocapsules selon la revendication 1 ou 2, **caractérisées par le fait que** les éther-diols sont des di-, tri- ou tétra-(alkylène en C₂₋₄)-glycols tels que le di-, tri ou tétraéthylèneglycol, le di-, tri- ou tétrapropylèneglycol ou le di-, tri- ou tétrabutylèneglycol.

4. Nanocapsules selon l'une quelconque des revendications précédentes, **caractérisées par le fait que** les triols sont choisis parmi le glycérol, le triméthyloléthane et le triméthylolpropane.

5. Nanocapsules selon l'une quelconque des revendications précédentes, **caractérisées par le fait que** les motifs dérivés des triols représentent au plus 5 % en moles de l'ensemble des motifs dérivés des diols et triols.

6. Nanocapsules selon l'une quelconque des revendications précédentes, **caractérisées par le fait que** lesdits polyesters formant l'enveloppe sont des poly(éthylène adipate) ou des poly(butylène adipate) linéaires.

7. Nanocapsules selon l'une des revendications précédentes **caractérisées par le fait que** les polyesters formant l'enveloppe ont une masse molaire moyenne en poids mesurée par chromatographie de perméation de gel comprise entre 2000 et 50 000, de préférence entre 5000 et 15 000.

8. Nanocapsules selon l'une quelconque des revendications précédentes, **caractérisées par le fait que** l'enveloppe polymérique est entourée d'un enrobage lamellaire ayant une structure organisée en un ou plusieurs feuillet(s) constitué(s) chacun d'une double couche de molécules amphiphiles également appelées agent d'enrobage.

9. Nanocapsules selon la revendication 8, **caractérisées par le fait que** ledit agent d'enrobage est choisi parmi les phospholipides, notamment la lécithine, les polycondensats d'oxyde de propylène et d'oxyde d'éthylène et les agents tensioactifs siliconés, capables de former des structures lamellaires.

10. Nanocapsules selon l'une quelconque des revendications précédentes, **caractérisées par le fait qu'**elles ont une taille moyenne comprise entre 50 nm et 800 nm, de préférence entre 100 nm et 300 nm.

11. Nanocapsules selon l'une quelconque des revendications précédentes, **caractérisées par** le fait les principes actifs lipophiles encapsulés sont choisis parmi les agents émollients, les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les piégeurs de radicaux libres, les agents hydratants et les vitamines.

12. Nanocapsules selon la revendication 11, **caractérisées par le fait que** le principe actif lipophile encapsulé est choisi parmi les principes actifs lipophiles sensibles aux conditions physico-chimiques environnantes telles que la température, le pH, la lumière ou la présence d'agents oxydants.

13. Nanocapsules selon la revendication 12, **caractérisées par le fait que** le principe actif lipophile est choisi parmi les vitamines telles que la vitamine A, la vitamine E, la vitamine D et la vitamine F et les esters et dérivés de celles-ci, les carotènes tels que le β-carotène et les dérivés de ceux-ci tels que le lycopène, et l'acide salicylique et les dérivés de celui-ci, en particulier les acides n-octanoyl-5-salicylique, n-décanoyl-5-salicylique, n-dodécanoyl-5-salicylique, n-octyl-5-salicylique, n-heptyloxy-5-salicylique et n-heptyloxy-4-salicylique..

14. Nanocapsules selon la revendication 13, **caractérisées par le fait que** le principe actif lipophile est le rétinol ou un ester en C₁₋₃₀, plus particulièrement en C₁₋₆, de celui-ci, tel que l'acétate de rétinol ou le propionate de rétinol.

15. Composition cosmétique ou dermatologique, **caractérisée par le fait qu'**elle contient, dans un support physiologiquement acceptable, les nanocapsules à base de poly(alkylène adipate) selon l'une quelconque des revendications 1 à 14.

16. Composition cosmétique ou dermatologique selon la revendication 15, **caractérisée par le fait que** la fraction des nanocapsules est comprise entre 0,1 et 30 % en poids et de préférence entre 0,5 et 15 % en poids rapporté au poids total de la composition.

17. Composition cosmétique ou dermatologique selon la revendication 15 ou 16, **caractérisée par le fait qu'**elle contient en outre des adjuvants cosmétiques et/ou pharmaceutiques tels que des corps gras, de la vaseline, des agents régulateurs de pH, des conservateurs, des agents épaississants, des colorants ou des parfums.

18. Composition cosmétique ou dermatologique selon l'une quelconque des revendications 15 à 17, **caractérisée par le fait qu'**elle se présente sous forme d'un sérum, d'une lotion, d'un gel aqueux, hydroalcoolique ou huileux, d'une émulsion eau-dans-huile ou huile-dans-eau, ou encore sous forme d'une dispersion aqueuse de vésicules lipidiques constituées de lipides ioniques ou non-ioniques ou d'un mélange de ceux-ci, lesquelles vésicules renferment ou non une phase huileuse.

19. Procédé de préparation des nanocapsules selon l'une quelconque des revendications 1 à 14, consistant
- à dissoudre un polymère, une phase lipidique formant ou contenant un principe actif et éventuellement un agent d'enrobage dans un solvant organique miscible à l'eau approprié,
- à préparer une solution aqueuse d'un agent tensioactif approprié,
- à verser la phase organique dans la phase aqueuse tout en agitant modérément celle-ci,
- puis à évaporer la phase organique et, éventuellement, une partie de la phase aqueuse,
procédé de préparation **caractérisé par le fait que** le polymère utilisé dans la première étape est un poly(alkylène adipate) décrit dans l'une quelconque des revendications 1 à 7.

## Patentansprüche

1. Nanokapseln, die bestehen aus
- einem Lipidkern, der einen lipophilen Wirkstoff bildet oder enthält, und
- einer wasserunlöslichen kontinuierlichen polymeren Hülle,
**dadurch gekennzeichnet, dass** die polymere Hülle mindestens einen geradkettigen oder verzweigten Polyester vom Typ eines Poly(alkylenadipats) aufweist, der von Adipinsäure abgeleitete Einheiten und von einem oder mehreren Alkandiolen und/oder einem oder mehreren Etherdiolen und/oder einem oder mehreren Triolen abgeleitete Einheiten enthält.

2. Nanokapseln nach Anspruch 1, **dadurch gekennzeichnet, dass** die Alkandiole geradkettige oder verzweigte C₂₋₆-Alkandiole sind, die unter Ethylenglykol, Propylenglykol, 1,3-Propandiol,
1,4-Butandiol, 1,5-Pentandiol, 1,6-Hexandiol und Neopentylglykol ausgewählt sind.

3. Nanokapseln nach Anspruch 1 oder 2, **dadurch gekennzeichnet, dass** die Etherdiole Di-, Tri- oder Tetra-(C₂₋₄-alkylen)-alykole wie etwa Di-, Tri- oder Tetraethylenglykol, Di-, Tri- oder Tetrapropylenglykol oder Di-, Tri- oder Tetrabutylenglykol sind.

4. Nanokapseln nach einem der vorhergehenden Ansprüche, **dadurch gekennzeichnet, dass** die Triole unter Glycerin, Trimethylolethan und Trimethylolpropan ausgewählt sind.

5. Nanokapseln nach einem der vorhergehenden Ansprüche, **dadurch gekennzeichnet, dass** die von Triolen abgeleiteten Einheiten höchstens 5 Mol-%. der Gesamtheit der von Diolen und Triolen abgeleiteten Einheiten darstellen.

6. Nanokapseln nach einem der vorhergehenden Ansprüche, **dadurch gekennzeichnet, dass** die Polyester, welche die Hülle bilden, geradkettige Poly(ethylenadipate) oder Poly(butylenadipate) sind.

7. Nanokapseln nach einem der vorhergehenden Ansprüche, **dadurch gekennzeichnet, dass** die Polyester, welche die Hülle bilden, eine durch Gelpermeationschromatographie bestimmte mittlere gewichtsbezogene Molekülmasse von 2000 bis 50 000 und bevorzugt 5000 bis 15 000 aufweisen.

8. Nanokapseln nach einem der vorhergehenden Ansprüche, **dadurch gekennzeichnet, dass** die polymere Hülle von einem lamellaren Überzug mit einer organisierten Struktur aus einer oder mehreren Schichten umgeben ist, die jeweils aus einer Doppelschicht von amphiphilen Molekülen bestehen, die auch als Überzugsmittel bezeichnet werden.

9. Nanokapseln nach Anspruch 8, **dadurch gekennzeichnet, dass** das Überzugsmittel unter Phospholipiden, insbesondere Lecithin, Polykondensationsprodukten von Propylenoxid und Ethylenoxid und Siliconhaltigen grenzflächenaktiven Mitteln ausgewählt ist, die dazu befähigt sind, lamellare Strukturen auszubilden.

10. Nanokapseln nach einem der vorhergehenden Ansprüche, **dadurch gekennzeichnet, dass** sie eine mittlere Größe von 50 bis 800 nm und bevorzugt 100 bis 300 nm besitzen.

11. Nanokapseln nach einem der vorhergehenden Ansprüche, **dadurch gekennzeichnet, dass** die eingekapselten lipophilen Wirkstoffe ausgewählt sind unter Emollientien, entzündungshemmenden Mitteln, antibakteriellen Mitteln, Mitteln gegen Pilze, Antivirusmitteln, Antiseborrhoemitteln, Antiaknemitteln, Keratolytika, Antihistaminen, Anästhetika, Mitteln zur Narbenbildung, Modifikatoren der Pigmentierung, Sonnenfiltern, Radikalfängern für freie Radikale, Hydratisierungsmitteln und Vitaminen.

12. Nanokapseln nach Anspruch 11, **dadurch gekennzeichnet, dass** der eingekapselte lipophile Wirkstoff ausgewählt ist unter lipophilen Wirkstoffen, die gegenüber physikalisch-chemischen Umgebungsbedingungen wie Temperatur, pH-Wert, Licht oder Vorliegen von Oxidationsmitteln empfindlich sind.

13. Nanokapseln nach Anspruch 12, **dadurch gekennzeichnet, dass** der lipophile Wirkstoff ausgewählt ist unter Vitaminen, wie Vitamin A, Vitamin E, Vitamin D und Vitamin F, sowie Estern und Derivaten davon, Carotinen, wie _-Carotin und Derivaten davon, wie Lycopin, sowie Salicylsäure und ihren Derivaten, insbesondere 5-n-Octanoylsalicylsäure, 5-n-Decanoylsalicylsäure, 5-n-Dodecanoylsalicylsäure, 5-n-Octylsalicylsäure, 5-n-Heptyloxysalicylsäure und 4-n-Heptyloxysalicylsäure.

14. Nanokapseln nach Anspruch 13, **dadurch gekennzeichnet, dass** der lipophile Wirkstoff Retinol oder ein C₁₋₃₀-Ester und insbesondere ein C₁₋₆-Ester davon ist, wie zum Beispiel Retinolacetat oder Retinolpropionat.

15. Kosmetische oder dermatologische Zusammensetzung, **dadurch gekennzeichnet, dass** sie in einem physiologisch akzeptablen Träger Nanokapseln auf der Basis von Poly(alkylenadipat) nach einem der Ansprüche 1 bis 14 enthält.

16. Kosmetische oder dermatologische Zusammensetzung nach Anspruch 15, **dadurch gekennzeichnet, dass** der Anteil der Nanokapseln 0,1 bis 30 Gew.-% und vorzugsweise 0,5 bis 15 Gew.-%, bezogen auf das Gesamtgewicht der Zusammensetzung, beträgt.

17. Kosmetische oder dermatologische Zusammensetzung nach Anspruch 15 oder 16, **dadurch gekennzeichnet, dass** sie ferner kosmetische und/oder pharmazeutische Hilfsstoffe, wie Fettkörper, Vaseline, pH-Regulatoren, Konservierungsmittel, Verdickungsmitteln, Färbemittel oder Parfums, enthält.

18. Kosmetische oder dermatologische Zusammensetzung nach einem der Ansprüche 15 bis 17, **dadurch gekennzeichnet, dass** sie in Form eines Serums, einer Lotion, eines wässerigen Gels, eines wässerig-alkoholischen Gels oder eines öligen Gels, einer
Wasser-in-Öl-Emulsion oder einer Öl-in-Wasser-Emulsion oder in Form einer wässerigen Dispersion von Lipidvesikeln vorliegt, die aus ionischen oder nichtionischen Lipiden oder einem Gemisch davon aufgebaut sind, wobei die Vesikel gegebenenfalls eine Ölphase einschließen.

19. Verfahren zur Herstellung der Nanokapseln nach einem der Ansprüche 1 bis 14, das aus folgenden Stufen besteht:
- Lösen eines Polymers, einer Lipidphase, die einen Wirkstoff bildet oder enthält, sowie gegebenenfalls eines Überzugsmittels in einem geeigneten, mit Wasser mischbaren organischen Lösungsmittel,
- Herstellen einer wässerigen Lösung eines geeigneten grenzflächenaktiven Mittels,
- Eingießen der organischen Phase in die wässerige Phase unter mäßigem Rühren dieser Phase und
- anschließendes Abdampfen der organischen Phase und gegebenenfalls eines Teils der wässerigen Phase,
**dadurch gekennzeichnet, dass** das in der ersten Stufe eingesetzte Polymer ein Poly(alkylenadipat) ist, das in einem der Ansprüche 1 bis 7 beschrieben ist.

## Claims

1. Nanocapsules consisting
- of a lipid centre forming or containing a lipophilic active principle, and
- of a water-insoluble continuous polymeric envelope,
**characterized in that** the said polymeric envelope comprises at least one linear or branched polyester of poly(alkylene adipate) type comprising units derived from adipic acid and units derived from one or more alkanediols and/or from one or more ether-diols and/or from one or more triols.

2. Nanocapsules according to Claim 1, **characterized in that** the alkanediols are C₂-C₆ alkanediols with a linear or branched chain, chosen from ethylene glycol, propylene glycol, 1,3-propanediol, 1,4-butanediol, 1,5-pentanediol, 1,6-hexanediol and neopentyl glycol.

3. Nanocapsules according to Claim 1 or 2, **characterized in that** the ether-diols are di-, tri- or tetra(C₂-C₄ alkylene) glycols, such as di-, tri- or tetraethylene glycol, di-, tri- or tetrapropylene glycol or di-, tri- or tetrabutylene glycol.

4. Nanocapsules according to any one of the preceding claims, **characterized in that** the triols are chosen from glycerol, trimethylolethane and trimethylolpropane.

5. Nanocapsules according to any one of the preceding claims, **characterized in that** the units derived from triols represent not more than 5 mol% of the total amount of units derived from diols and triols.

6. Nanocapsules according to any one of the preceding claims, **characterized in that** the said polyesters forming the envelope are linear poly(butylene adipates) or poly(ethylene adipates).

7. Nanocapsules according to one of the preceding claims, **characterized in that** the polyesters forming the envelope have a weight-average molar mass, measured by gel permeation chromatography, of between 2000 and 50,000, preferably between 5000 and 15,000.

8. Nanocapsules according to any one of the preceding claims, **characterized in that** the polymeric envelope is surrounded by a lamellar coating which has a structure organized as one or more lamella(e) each consisting of a double-layer of amphiphilic molecules also referred to as coating agent.

9. Nanocapsules according to Claim 8, **characterized in that** the said coating agent is chosen from phospholipids, in particular lecithin, polycondensates of propylene oxide and of ethylene oxide, and silicone surfactants, which are capable of forming lamellar structures.

10. Nanocapsules according to any one of the preceding claims, **characterized in that** they have an average size of between 50 nm and 800 nm, preferably between 100 nm and 300 nm.

11. Nanocapsules according to any one of the preceding claims, **characterized in that** the encapsulated lipophilic active principles are chosen from emollients, anti-inflammatory agents, antibacterial agents, antifungal agents, antiviral agents, anti-seborrhoeic agents, anti-acne agents, keratolytic agents, anti-histaminic agents, anaesthetics, cicatrizing agents, pigmentation modifiers, sunscreens, free-radical scavengers, moisturizers and vitamins.

12. Nanocapsules according to Claim 11, **characterized in that** the encapsulated lipophilic active principle is chosen from lipophilic active principles which are sensitive to the surrounding physicochemical conditions such as the temperature, pH, light or the presence of oxidizing agents.

13. Nanocapsules according to Claim 12, **characterized in that** the lipophilic active principle is chosen from vitamins such as vitamin A, vitamin E, vitamin D and vitamin F, or esters or derivatives thereof, carotenes such as β-carotene or derivatives thereof, for instance lycopene, and salicylic acid or derivatives thereof, in particular 5-n-octanoylsalicylic acid, 5-n-decanoylsalicylic acid, 5-n-dodecanoylsalicylic acid, 5-n-octylsalicylic acid, 5-n-heptyloxysalicylic acid and 4-n-heptyloxysalicylic acid.

14. Nanocapsules according to Claim 13, **characterized in that** the lipophilic active principle is retinol or a C₁-C₃₀, more particularly C₁-C₆, ester thereof, such as retinyl acetate or retinyl propionate.

15. Cosmetic or dermatological composition, **characterized in that** it contains, in a physiologically acceptable support, the nanocapsules based on poly(alkylene adipate) according to any one of Claims 1 to 14.

16. Cosmetic or dermatological composition according to Claim 15, **characterized in that** the fraction of nanocapsules is between 0.1 and 30% by weight and preferably between 0.5 and 15% by weight, relative to the total weight of the composition.

17. Cosmetic or dermatological composition according to Claim 15 or 16, **characterized in that** it also contains cosmetic and/or pharmaceutical adjuvants, such as fatty substances, petroleum jelly, pH regulators, preserving agents, thickeners, dyes or fragrances.

18. Cosmetic or dermatological composition according to any one of Claims 15 to 17, **characterized in that** it is in the form of an aqueous, aqueous-alcoholic or oily serum, lotion or gel, a water-in-oil or oil-in-water emulsion or alternatively in the form of an aqueous dispersion of lipid vesicles consisting of ionic or nonionic lipids or of a mixture thereof, these vesicles containing or not containing an oily phase.

19. Process for preparing the nanocapsules according to any one of Claims 1 to 14, which consists
- in dissolving a polymer, a lipid phase forming or containing an active principle, and optionally a coating agent, in a suitable water-miscible organic solvent,
- in preparing an aqueous solution of a suitable surfactant,
- in adding the organic phase to the aqueous phase with moderate stirring thereof,
- and then in evaporating the organic phase and, possibly, some of the aqueous phase,
this preparation process being **characterized in that** the polymer used in the first step is a poly(alkylene adipate) described in any one of Claims 1 to 7.
